# EUROPÄISCHE PATENTSCHRIFT

(11) **EP 2 709 731 B1**
(45) Veröffentlichungstag und Bekanntmachung des Hinweises auf die Patenterteilung: **08.11.2017**
(21) Anmeldenummer: 12719577.4
(22) Anmeldetag: 25.04.2012
(51) Int. Cl.: A61Q 19/04, A61K 8/97, A61K 36/185, A61Q 17/04, A61Q 19/08

(54) **EXTRAKTE AUS DARLINGTONIA CALIFORNICA**
EXTRACTS OF DARLINGTONIA CALIFORNICA
EXTRAITS DE DARLINGTONIA CALIFORNICA

(30) Priorität: 18.05.2011 DE 102011101875
(43) Veröffentlichungstag der Anmeldung: 26.03.2014
(73) Patentinhaber: Merck Patent GmbH, 64293 Darmstadt (DE)
(72) Erfinder: WIRTH, Corinna, 64283 Darmstadt (DE)
(86) Internationale Anmeldenummer: PCT/EP2012/001770
(87) Internationale Veröffentlichungsnummer: WO 2012/156024

(56) Entgegenhaltungen:
- WO-A1-99/42115
- DE-A1- 3 844 244
- RANDALL J. BAYER ET AL: "Phylogenetic Relationships in Sarraceniaceae Based on rbcL and ITS Sequences", SYSTEMATIC BOTANY, Bd. 21, Nr. 2, 1. Juni 1996 (1996-06-01), Seiten 121-134, XP055085914, ISSN: 0363-6445, DOI: 10.2307/2419743
- A. M. ELLISON ET AL: "The cost of carnivory for Darlingtonia californica (Sarraceniaceae): evidence from relationships among leaf traits", AMERICAN JOURNAL OF BOTANY, Bd. 92, Nr. 7, 1. Juli 2005 (2005-07-01), Seiten 1085-1093, XP055085905, ISSN: 0002-9122, DOI: 10.3732/ajb.92.7.1085
- DATABASE BIOSIS [Online] BIOSCIENCES INFORMATION SERVICE, PHILADELPHIA, PA, US; 1. Januar 1985 (1985-01-01), RONDINA R V D ET AL: "STANDARD METHOD FOR THE SYSTEMATIC PREPARATION AND FRACTIONATION OF PLANT EXTRACTS TO TRACE THEIR PHARMACOLOGICAL ACTIVITY AND FOR CHEMICAL STUDY", XP002212492, gefunden im BIOSIS Database accession no. PREV198681046341

## Beschreibung

Die vorliegende Erfindung betrifft einen Extrakt aus Pflanzenteilen von *Darlingtonia californica* zur Verwendung als Selbstbräunungsmittel, sowie Zubereitungen enthaltend einen Extrakt aus Pflanzenteilen von *Darlingtonia californica* und deren Herstellung.

Der Trend weg von der vornehmen Blässe hin zur "gesunden, sportlich gebräunten Haut" ist seit Jahren ungebrochen. Um einen gebräunten Teint zu erzielen, setzen die Menschen ihre Haut der Sonnenstrahlung aus, da diese eine Pigmentierung durch eine Melaninbildung hervorruft. Die UV-Strahlung des Sonnenlichtes hat jedoch auch eine schädigende Wirkung auf die Haut. Neben der akuten Schädigung (Sonnenbrand) treten Langzeitschäden bei übermäßiger Bestrahlung mit Licht aus dem UVB-Bereich (Wellenlänge 280-320 nm) auf, wie beispielsweise ein erhöhtes Risiko, an Hautkrebs zu erkranken. Die übermäßige Einwirkung der UVB- und UVA-Strahlung (Wellenlänge: 320-400 nm) erzeugt hochreaktive Radikalspezies, die sich auch nach Beendigung der Bestrahlung weiter vermehren und als deren Folge es zu Faltenbildung und Hautalterung kommt.

Den natürlichen Schutz vor den negativen Folgen der Sonnenstrahlung bietet die Bräunung (Pigmentierung) der Haut. Die Epidermis enthält in ihrer untersten Schicht, der Basalschicht, neben den Basalzellen einzelne pigmentbildende Zellen, die Melanocyten. Durch UV-Licht wird in diesen Zellen die Produktion von Melanin angeregt, das in die Kerantinocyten (Hornzellen) transportiert und dort als braune Hautfarbe sichtbar wird. Melanin schützt die Zellkerne vor weiterer Bestrahlung und den davon verursachten negativen Auswirkungen auf die Zell-DNA.

Je nach chemischer Zusammensetzung der biochemisch gebildeten Pigmente wird zwischen dem bräunlich-schwarzen Eumelanin und dem rötlich-gelben Pheomelanin unterschieden. Der beobachtete Hautfarbton wird vom Verhältnis dieser beiden Melaninarten bestimmt.
Diese von der Aminosäure Tyrosin ausgehende Pigmentbildung wird überwiegend durch UVB-Strahlung initiiert und als "indirekte Pigmentierung" bezeichnet. Ihre Entwicklung läuft über mehrere Tage; die so erhaltene Sonnenbräune besteht über einige Wochen. Bei der "Direkt-Pigmentierung", die mit der Sonnenbestrahlung einsetzt, werden vorwiegend farblose Melanin-Vorstufen durch UVA-Strahlung zu dunkel gefärbtem Melanin oxidiert. Da diese Oxidierung reversibel ist, führt sie zu einer nur kurz anhaltenden Hautbräunung.

Eine künstliche Bräunung der Haut lässt sich äußerlich mit Hilfe von Schminke und oral durch Einnahme von Carotinoiden erzeugen.

Weitaus beliebter jedoch ist die künstliche Bräunung der Haut, welche sich durch Auftragen von sogenannten Selbstbräunern erzielen lässt.
Diese Verbindungen weisen als chemisches Strukturmerkmal Keto- bzw. Aldehydgruppen in Nachbarschaft zu Alkoholfunktionen auf und gehören überwiegend zur Substanzklasse der Zucker. Besonders häufig eingesetzte Selbstbräunungssubstanzen sind 1,3-Dihydroxyaceton (DHA), das in einer Menge von 700t/a verwendet wird, und Erythrulose.

Klassische Selbstbräuner können mit den Proteinen und Aminosäuren der Hornschicht der Haut im Sinne einer Maillard-Reaktion oder über eine Michael Addition umgesetzt werden, wobei über einen noch nicht vollständig aufgeklärten Reaktionsweg Polymerisate entstehen, die der Haut einen bräunlichen Farbton verleihen. Diese Reaktion ist nach etwa 4 bis 6 Stunden abgeschlossen. Die so erzielte Bräune ist nicht abwaschbar und wird erst mit der normalen Hautabschuppung entfernt.

Diese Farbprodukte besitzen allerdings selbst keine UV-absorbierenden Eigenschaften, sodass bei Sonnenexposition ein zusätzlicher Sonnenschutz (Bekleidung, Hut, UV-Filter) erforderlich wird.
Im Gegensatz zu "sonnengebräunter" Haut ist die so gebräunte Haut nicht gegen Sonnenbrand geschützt.
Es besteht daher ein Bedarf nach dermatologisch verträglichen hautfärbenden Substanzen, die sich zum Einsatz in kosmetischen und/oder dermatologischen Zubereitungen oder Medizinprodukten eignen und die die natürliche Bräunung der Haut durch Steigerung der Melaninsynthese verstärken und gleichzeitig einen besseren Hauteigenschutz bzw. Sonnenschutz, insbesondere gegen UVB-Strahlung, ermöglichen.
Die der vorliegenden Erfindung zugrunde liegende Aufgabe bestand daher in der Bereitstellung von neuen Selbstbräunungsmitteln mit verbesserten Eigenschaften.
Überraschenderweise wurde nun festgestellt, dass sich Extrakte aus Pflanzenteilen von *Darlingtonia californica* als Selbstbräunungsmittel eignen.
Im Sinne der Erfindung wird der Begriff Selbstbräunungswirkstoff synonym zu Selbstbräunungssubstanz, Selbstbräuner oder Selbstbräunersubstanz verwendet.

*Darlingtonia californica* (Kobralilie) ist eine fleischfressende Pflanze, die als einzige Art der Gattung *Darlingtonia* zur Familie der Schlauchpflanzengewächse (Sarraceniaceae) gezählt wird.

Extrakte dieser Pflanze sind im Stand der Technik bekannt: WO99/42115 A1 beschreibt die Verwendung eines Press-Saftes, Verdauungssaftes oder Extraktes von fleischfressenden Pflanzen, darunter Darlingtonia, zur Herstellung eines Medikaments zur Hemmung von Proteinkinasen.

EP 0249165 A2 beschreibt die Verwendung von Verdauungssäften von fleischfressenden Pflanzen wie *Darlingtonia* in Arzneimitteln zur Bekämpfung von malignen und chronischen Erkrankungen, die eine Veränderung der Zellstrukturen der Körperzellen direkt oder indirekt bewirken.
US 2007/0122492 A1 offenbart ein Verfahren zur Identifizierung von Pflanzenextrakten, die in der Lage sind, verschiedene extrazelluläre Proteasen wie die Matrix-Metalloproteasen (MMP) oder die humane Leukozytenelastase (HLE) zu inhibieren. Als mögliche zu untersuchende Pflanzen wird die Familie der Sarraceniaceae gelistet.
US 6350594 B1 beschreibt die Gewinnung von Pflanzengummis aus Pflanzenzellen in Suspensionskultur und deren Verwendung in der Kosmetik. Eine Liste möglicher Pflanzen enthält die Familie der Sarraceniaceae.

Ein erster Gegenstand der vorliegenden Erfindung ist ein Extrakt aus Pflanzenteilen von *Darlingtonia californica* zur Verwendung als Selbstbräunungsmittel. Prinzipiell können alle oberirdischen Teile der Pflanze für den Extrakt genutzt werden. Bevorzugt eingesetzte Pflanzenteile sind die Blätter. Die Blätter von *Darlingtonia californica* sind schlauchförmig ("Schlauchblätter") und dienen als Fallen für den Insektenfang.
Der Pflanzenextrakt aus *Darlingtonia californica* kann nach dem Fachmann bekannten Methoden hergestellt werden. Ein für die erfindungsgemäße Verwendung besonders gut geeigneter Pflanzenextrakt ist erhältlich durch a) Extraktion der Pflanzenteile von *Darlingtonia californica* mithilfe eines Lösungsmittels und b) Entfernen des Lösungsmittels.

Die Pflanzenteile können direkt zur Extraktion gemäß Schritt a) eingesetzt werden. Typischerweise werden die Pflanzenteile jedoch zunächst getrocknet und zerkleinert.
Die Trocknung kann beispielsweise an der Luft oder bevorzugt in einem Trockenschrank bei erhöhter Temperatur, bevorzugt zwischen 30 und 50°C, besonders bevorzugt bei etwa 40°C erfolgen. Ausserdem kann die Trocknung auch unter reduziertem Druck durchgeführt werden.
Das Zerkleinern kann mittels gängiger Methode, beispielsweise mithilfe von Schneidwerkzeugen wie Messern oder Scheren oder mithilfe eines Mixgeräts erfolgen.

Anschließend werden die Pflanzenteile extrahiert. Die Extraktion wird mithilfe von Methoden durchgeführt, die dem Fachmann bekannt sind. Dafür werden die Pflanzenteile mit einem Lösungsmittel versetzt.
Bevorzugt wird ein Lösungsmittel ausgewählt aus Wasser, organischen Lösungsmitteln oder Mischungen hiervon eingesetzt. Beispiele für organische Lösungsmittel sind Methanol, Ethanol, 1-Propanol, 2-Propanol, Acetonitril, Aceton oder Ethylacetat.
Lösungsmittel-Mischungen aus Wasser und organischen Lösungsmitteln enthalten bevorzugt 10-90 Vol.-% Wasser, besonders bevorzugt 30-70 Vol.-% Wasser.
In einer bevorzugten Ausführungsform der vorliegenden Erfindung ist das Lösungsmittel ausgewählt aus Wasser oder Alkohol. In einer besonders bevorzugten Ausführungsform wird ein Alkohol, insbesondere Ethanol, als Lösungsmittel verwendet.

Die Extraktion wird typischerweise bei Temperaturen zwischen 20 und 90°C durchgeführt, bevorzugt zwischen 50 und 90°C, besonders bevorzugt bei 70°C.

In Schritt b) wird das Lösungsmittel entfernt. Dies erfolgt typischerweise durch Filtration.

Der so erhaltene Extrakt kann optional weiter aufgereinigt werden. Hierfür können dem Fachmann gängige Methoden eingesetzt werden, beispielsweise chromatographische Methoden wie Festphasenadsorption mit anschließender stufenweiser Desorption mittels geeigneter Lösemittel. Die Chromatographie kann auch im Gegenstrom geführt werden. Eine Aufkonzentration und Anreicherung kann auch mittels überkritischer Gase oder ionischer Flüssigkeiten erfolgen. Es ist außerdem möglich, durch Flüssig/Flüssig Extraktion eine Anreicherung von unerwünschten Bestandteilen zu erreichen oder auch durch Wechseln des Lösungsmittels mit anschließender Filtration unerwünschte Bestandteile zu entfernen.

Optional kann der erhaltene Extrakt anschließend in einem Schritt c) aufkonzentriert und/oder getrocknet werden.
Dies kann mit dem Fachmann gängigen Methoden erfolgen. Die Aufkonzentration erfolgt beispielsweise unter Verwendung eines Rotationsverdampfers oder eines Fallfilmverdampfers, und kann auch unter reduziertem Druck erfolgen. Die Trocknung des Extrakts kann beispielsweise mithilfe von Sprühtrocknung oder Gefriertrocknung erfolgen. Bevorzugt wird der Extrakt dabei komplett zur Gewichtskonstanz getrocknet.

Extrakte aus Pflanzenteile von *Darlingtonia californica* weisen sehr gute Bräunungseigenschaften auf. Es wird vermutet, dass die hohe Bräunungsaktivität der erfindungsgemäßen Extrakte, ohne an diese Theorie gebunden zu sein, auf eine Kombination verschiedener aktiver Komponenten in den Extrakten zurückzuführen ist. Die bräunende Wirkung des Extrakts ist dabei besonders gut, wenn er nach der oben beschriebenen Methode und insbesondere nach ihren bevorzugten Ausführungsformen hergestellt wird. Die erfindungsgemäßen Extrakte eignen sich hervorragend zur Einarbeitung in kosmetische oder dermatologische Zubereitungen, insbesondere auch wegen ihrer neutralen Farbe, die die Zubereitung nicht auffällig färbt. Die Herstellung des Extraktes ist dabei sehr umweltfreundlich, da nur geringe Mengen Lösungsmittel eingesetzt werden müssen und keine weiteren Chemikalien notwendig sind.

Erfindungsgemäß bevorzugt ist der Extrakt zur Verwendung zur Steigerung der Melaninsynthese, Verbesserung des Melanintransports und/oder Verbesserung der Verteilung von Melanin in suprabasalen Schichten.

Die erfindungsgemäßen Extrakte steigern die Melaninsynthese und verbessern den Melanintransport von den Melanocyten zu den Keratinocyten. Dies wirkt sich auf die Farbe der Haut aus und bewirkt einen Bräunungseffekt.

Ein weiterer Gegenstand der vorliegenden Erfindung ist eine Zubereitung enthaltend einen Extrakt aus Pflanzenteilen von *Darlingtonia californica* wie zuvor beschrieben, dadurch gekennzeichnet, dass mindestens eine weitere Selbstbräunungssubstanz enthalten ist. Bei den Zubereitungen handelt es sich dabei üblicherweise um topisch anwendbare Zubereitungen, beispielsweise kosmetische oder dermatologische Formulierungen oder Medizinprodukte. Die Zubereitungen enthalten in diesem Fall einen kosmetisch oder dermatologisch geeigneten Träger und je nach gewünschtem Eigenschaftsprofil optional weitere geeignete Inhaltsstoffe. Handelt es sich um pharmazeutische Zubereitungen, so enthalten die Zubereitungen in diesem Fall einen pharmazeutisch verträglichen Träger und optional weitere pharmazeutische Wirkstoffe.
Im Sinne der vorliegenden Erfindung wird neben dem Begriff Zubereitung gleichbedeutend auch der Begriff Formulierung verwendet.
Topisch anwendbar bedeutet im Sinne der Erfindung, dass die Zubereitung äußerlich und örtlich angewendet wird, d.h. dass die Zubereitung geeignet sein muss, um beispielsweise auf die Haut aufgetragen werden zu können.
Die Zubereitungen können die genannten notwendigen oder optionalen Bestandteile umfassen oder enthalten, daraus im wesentlichen oder daraus bestehen. Alle Verbindungen oder Komponenten, die in den Zubereitungen verwendet werden können, sind entweder bekannt und käuflich erwerbbar oder können nach bekannten Verfahren synthetisiert werden.
Bevorzugt handelt es sich um eine kosmetische oder pharmazeutische Zubereitung; besonders bevorzugt handelt es sich um eine kosmetische Zubereitung.

Die erfindungsgemäßen Zubereitungen enthalten bevorzugt 0,01 bis 99 Gew.% des Extrakts aus *Darlingtonia californica,* bezogen auf das Gesamtgewicht der Zubereitung. Bevorzugt wird eine Menge von 0,05 bis 30 Gew.% eingesetzt, besonders bevorzugt von 0,1 bis 10 Gew.%. Dabei bereitet es dem Fachmann keinerlei Schwierigkeiten die Mengen abhängig von der beabsichtigten Wirkung der Zubereitung entsprechend auszuwählen. Erfindungsgemässe Zubereitungen enthalten mindestens eine weitere Selbstbräunungssubstanz als weiteren Inhaltsstoff. Dabei kann es sich sowohl um einen Selbstbräuner handeln, der mit den Aminosäuren der Haut im Sinne einer Maillard-Reaktion oder über eine Michael-Addition reagiert, als auch um einen sogenannten Melanogenese-Promotor oder Propigmentierungswirkstoff, der die natürliche Bräunung der Haut fördert.
Als vorteilhafte Selbstbräunungssubstanzen können unter anderem eingesetzt werden: 1,3-Dihydroxyaceton, Glycerolaldehyd, Hydroxymethylglyoxal, γ-Dialdehyd, Erythrulose, 6-Aldo-D-Fructose, Ninhydrin, 5-Hydroxy-1,4-naphtochinon (Juglon) oder 2-Hydroxy-1,4-naphtochinon (Lawson). Ganz besonders bevorzugt ist 1,3-Dihydroxyaceton, Erythrulose oder deren Kombination.
Propigmentierungsstoffe können prinzipiell alle dem Fachmann bekannten Wirkstoffe sein. Beispiele hierfür sind Glycyrrhetinsäure, Melanocytenstimulierendes Hormon (alpha-MSH), Peptidanaloga, Thymidin-Dinucleotide, L-Tyrosin und dessen Ester oder bicyclische Monoterpendiole (beschrieben in Brown et al., Photochemistry and Photobiology B: Biology 63 (2001) 148-161).
Bevorzugt ist die mindestens eine weitere Selbstbräunungssubstanz in der Zubereitung in einer Menge von 0,01 bis 20 Gew.-%, besonders bevorzugt in einer Menge von 0,5 bis 15 Gew.-% und ganz besonders bevorzugt in einer Menge von 1 bis 8 Gew.-%, bezogen auf die Gesamtmenge der Zubereitung, enthalten.

Zubereitungen mit Selbstbräunereigenschaften, insbesondere solche, die Dihydroxyaceton enthalten, neigen bei der Anwendung auf der menschlichen Haut zu Fehlgerüchen, die vermutlich durch Abbauprodukte des Dihydroxyacetons selbst oder durch Produkte von Nebenreaktionen verursacht werden und die von den Anwendern teilweise als unangenehm empfunden werden. Es hat sich gezeigt, dass diese Fehlgerüche bei Verwendung von Formaldehydfängern und/oder Flavonoiden vermieden werden. Daher kann die erfindungsgemäße Zubereitung auch Formaldehydfänger sowie gegebenenfalls Flavonoide zur Verbesserung des Geruches enthalten.

Die erfindungsgemäße Zubereitung, welche eine weitere Selbstbräunungssubstanz und einen Extrakt aus Pflanzenteilen von *Darlingtonia californica* kombiniert, hat gegenüber einem Selbstbräunungsprodukt ohne Zugabe des erfindungsgemäßen Extrakts folgende Vorteile:
- Verlängerung der Bräunungs-Reaktion aufgrund der indirekten Bräunungs-Reaktion (UV-freie Bräunungsverlängerung),
- Verstärkung der Bräunungs-Reaktion,
- Vermeidung von ungleichmäßiger Bräunung durch ungeschicktes Auftragen,
- die erzielte Bräunung kommt der natürlichen Bräunung nahe,
- Verbesserung des Schutzes vor UV-Strahlung.

Die erfindungsgemäßen Zubereitungen können auch neben den erfindungsgemäßem Extrakten zusätzlich mindestens einen UV-Filter enthalten.

Organische UV-Filter, sogenannte hydrophile oder lipophile Sonnenschutzfilter, sind im UVA-Bereich und/oder UVB-Bereich und/oder IR und/oder VIS-Bereich (Absorber) wirksam. Diese Substanzen können insbesondere unter Zimtsäurederivaten, Salicylsäurederivaten, Campherderivaten, Triazinderivaten, β,β-Diphenylacrylatderivaten, p-Aminobenzoesäurederivaten sowie polymeren Filtern und Siliconfiltern, die in der Anmeldung WO-93/04665 beschrieben sind, ausgewählt sein.
Weitere Beispiele für organische Filter sind in der Patentanmeldung EP-A 0 487 404 angegeben. Im Folgenden werden die genannten UV-Filter meist nach der INCI-Nomenklatur benannt.

Insbesondere für eine Kombination geeignet sind:
para-Aminobenzoesäure und deren Derivate: PABA, Ethyl PABA, Ethyl dihydroxypropyl PABA, Ethylhexyl dimethyl PABA, z. B. vertrieben unter dem Namen "Escalol 507" von der Fa. ISP, Glyceryl PABA, PEG-25 PABA, z. B. vertrieben unter dem Namen "Uvinul P25" von der Fa. BASF.

Salicylate: Homosalate vetrieben unter dem Namen "Eusolex HMS" von der Fa. Merck; Ethylhexyl salicylate, z. B. vertrieben unter dem Namen "Neo Heliopan OS" von der Fa. Symrise, Dipropylene glycol salicylate, z. B. vertrieben unter dem Namen "Dipsal" von der Fa. Scher, TEA salicylate, z. B. vertrieben unter dem Namen "Neo Heliopan TS" von der Fa. Symrise.

β,β-Diphenylacrylate Derivate: Octocrylene, z. B. vertrieben unter dem Namen "Eusolex® OCR" von der Firma Merck", "Uvinul N539" von der Fa. BASF, Etocrylene, z. B. vertrieben unter dem Namen "Uvinul N35" von der BASF.

Benzophenone Derivate: Benzophenone-1, z. B. vertrieben unter dem Namen "Uvinul 400"; Benzophenone-2, z. B. vertrieben unter dem Namen "Uvinul D50" ; Benzophenone-3 oder Oxybenzone, z. B. vertrieben unter dem Namen "Uvinul M40";Benzophenone-4, z. B. vertrieben unter dem Namen "Uvinul MS40" ; Benzophenone-9, z. B. vertrieben unter dem Namen "Uvinul DS-49" von der Fa. BASF, Benzophenone-5, Benzophenone-6, z. B. vertrieben unter dem Namen "Helisorb 11" von der Fa. Norquay, Benzophenone-8, z. B. vertrieben unter dem Namen "Spectra-Sorb UV-24" von der Fa. American Cyanamid, Benzophenone-12 n-hexyl 2-(4-diethylamino-2-hydroxybenzoyl) benzoate oder 2-Hydroxy-4-methoxybenzophenon, vertrieben von der Fa. Merck, Darmstadt unter dem Namen Eusolex® 4360.

Benzylidencampher Derivate: 3-Benzylidenecamphor, z. B. vertrieben unter dem Namen "Mexoryl SD" von der Fa. Chimex, 4-Methylbenzylidenecamphor, z. B. vertrieben unter dem Namen "Eusolex 6300" von der Fa. Merck, Benzylidenecamphorsulfonsäure, z. B. vertrieben unter dem Namen "Mexoryl SL" von der Fa. Chimex, Camphor benzalkonium methosulfate, z. B. vertrieben unter dem Namen "Mexoryl SO" von der Fa. Chimex, Terephthalylidenedicamphorsulfonsäure, z. B. vertrieben unter dem Namen "Mexoryl SX" von der Fa Chimex, Polyacrylamidomethylbenzylidenecamphor vertrieben unter dem Namen "Mexoryl SW" von der Fa. Chimex.

Phenylbenzimidazol Derivate: Phenylbenzimidazolesulfonsäure, z. B. vertrieben unter dem Namen "Eusolex 232" von der Fa. Merck, Dinatrium phenyl dibenzimidazole tetrasulfonat, z. B. vertrieben unter dem Namen "Neo Heliopan AP" von der Fa. Symrise.

Phenylbenzotriazol Derivate: Drometrizole trisiloxane, z. B. vertrieben unter dem Namen "Silatrizole" von der Fa. Rhodia Chimie, Methylenebis(benzotriazolyl)tetramethylbutylphenol in fester Form, z. B. vertrieben unter dem Namen "MIXXIM BB/100" von der Fa. Fairmount Chemical, oder in mikronisierter Form als wässrige Dispersion, z. B. vertrieben unter dem Namen "Tinosorb M" von der Fa. BASF.

Triazin Derivate: Ethylhexyltriazone, z. B. vertrieben unter dem Namen "Uvinul T150" von der Fa. BASF, Diethylhexylbutamidotriazone, z. B. vertrieben unter dem Namen "Uvasorb HEB" von der Fa. Sigma 3V, 2,4,6-tris(diisobutyl 4'-aminobenzalmalonate)-s-triazine oder 2,4,6-Tris-(biphenyl)-1,3,5-triazine vertrieben als Tinosorb A2B von BASF, 2,2'-[6-(4-methoxyphenyl)-1,3,5-triazine-2,4-diyl]bis[5-(2-ethylhexyl)oxy]-phenol, vertrieben als Tinosorb S von BASF, N2,N4-bis[4-[5-(1,1-dimethylpropyl)-2-benzoxazolyl]phenyl]-N6-(2-ethylhexyl)-1,3,5-triazin-2,4,6-triamin vertrieben als Uvasorb K 2A von der Firma Sigma 3V, Bis(butylbenzoate) diaminotriazine aminopropyltrisiloxane (Mexoryl SBS).

Anthranilin Derivate: Menthyl anthranilate, z. B. vertrieben unter dem Namen "Neo Heliopan MA" von der Fa. Symrise.

Imidazol Derivate: Ethylhexyldimethoxybenzylidenedioxoimidazoline propionat.

Benzalmalonat Derivate: Polyorganosiloxane enthaltend funktionelle benzalmalonate Gruppen, wie z.B. Polysilicone-15, z. B. vertrieben unter dem Namen "Parsol SLX" von der Hoffmann LaRoche.

4,4-Diarylbutadien Derivate: 1,1-Dicarboxy(2,2'-dimethylpropyl)-4,4-diphenylbutadiene.

Benzoxazole Derivate: 2,4-bis[5-(1-dimethylpropyl)benzoxazol-2-yl(4-phenyl) imino]-6-(2-ethylhexyl)imino-1,3,5-triazine, z. B. vertrieben unter dem Namen Uvasorb K2A von der Fa. Sigma 3V und Mischungen dieses enthaltend.

Piperazinderivate wie beispielsweise die Verbindung 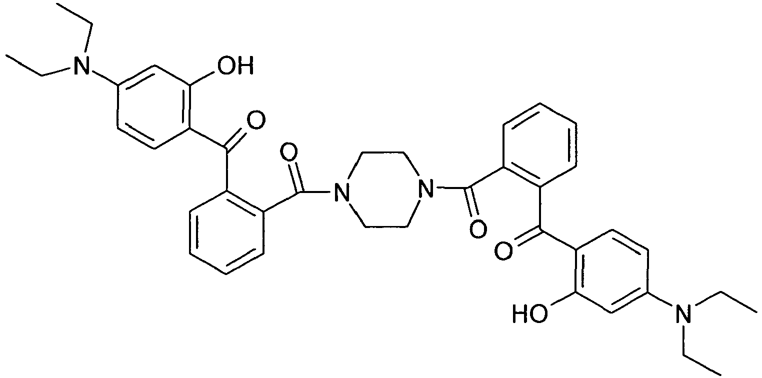 oder die UV-Filter der folgenden Strukturen 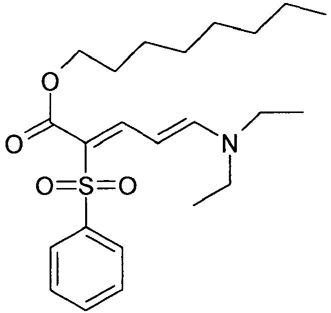 oder

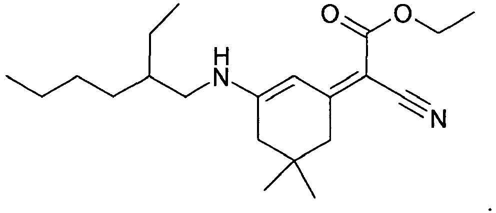

Es können auch UV-Filter auf Basis von Polysiloxancopolymeren mit einer statistischen Verteilung gemäß nachfolgender Formel verwendet werden, wobei z.B. a = 1,2; b= 58 und c=2,8 sind:

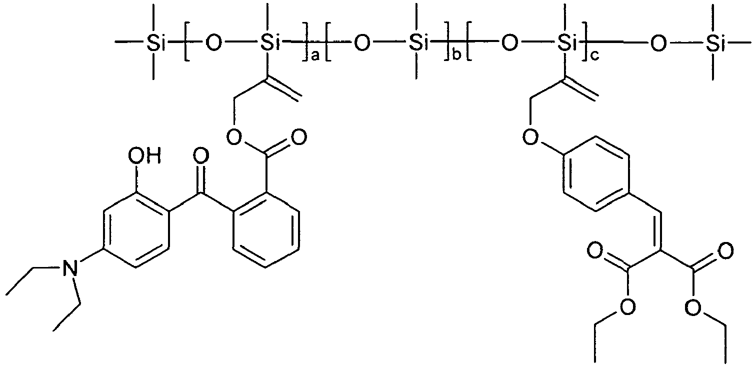

Die in der Liste aufgeführten Verbindungen sind nur als Beispiele aufzufassen. Selbstverständlich können auch andere UV-Filter verwendet werden.

Geeignete organischen UV-schützende Substanzen sind bevorzugt aus der folgenden Liste auszuwählen: Ethylhexyl salicylate, Phenylbenzimidazolesulfonic acid, Benzophenone-3, Benzophenone-4, Benzophenone-5, n-Hexyl 2-(4-diethylamino-2-hydroxybenzoyl)benzoate, 4-Methylbenzylidenecamphor, Terephthalylidenedicamphorsulfonic acid, Disodium phenyldibenzimidazoletetrasulfonate, Methylenebis(benzotriazolyl)tetramethylbutylphenol, Ethylhexyl Triazone, Diethylhexyl Butamido Triazone, Drometrizole trisiloxane, Polysilicone-15, 1,1-Dicarboxy(2,2'-dimethylpropyl)-4,4-diphenylbutadiene, 2,4-Bis[5-1 (dimethylpropyl)benzoxazol-2-yl(4-phenyl) imino]-6-(2-ethylhexyl)imino-1,3,5-triazine und Mischungen davon.

Diese organischen UV-Filter werden in der Regel in einer Menge von 0,01 Gewichtsprozent bis 20 Gewichtsprozent, vorzugsweise 1 Gew.-% bis 10 Gew.-%, in Formulierungen eingearbeitet.

Die Zubereitungen können neben den erfindungsgemäßen Extrakten sowie den gegebenenfalls organischen UV-Filtern, wie zuvor beschrieben, weitere anorganische UV-Filter, sogenannte partikuläre UV-Filter, enthalten.

Diese Kombinationen mit partikulären UV-Filtern sind sowohl als Pulver als auch als Dispersion oder Paste der folgenden Typen möglich.

Hierbei sind sowohl solche aus der Gruppe der Titandioxide wie z.B. gecoatetes Titandioxid (z.B. Eusolex® T-2000, Eusolex®T-AQUA, Eusolex®T-AVO, Eusolex®T-OLEO), Zinkoxide (z.B. Sachtotec®), Eisenoxide oder auch Ceroxide und/oder Zirkonoxide bevorzugt.

Ferner sind auch Kombinationen mit pigmentärem Titandioxid oder Zinkoxid möglich, wobei die Partikelgröße dieser Pigmente größer oder gleich 200 nm sind, beispielsweise Hombitan® FG oder Hombitan® FF-Pharma.

Weiter kann es bevorzugt sein, wenn die Zubereitungen anorganische UV-Filter enthalten, die mit üblichen Methoden, wie beispielsweise in Cosmetics & Toiletries, February 1990, Vol. 105, pp. 53 64 beschrieben, nachbehandelt wurden. Hierbei können eine oder mehrere der folgenden Nachbehandlungskomponenten gewählt sein: Amino Säuren, Bienenwachs, Fettsäuren, Fettsäurealkohole, anionische Tenside, Lecithin, Phospholipide, Natrium-, Kalium-, Zink-, Eisen- oder Aluminiumsalze von Fettsäuren, Polyethylene, Silikone, Proteine (besonders Collagen oder Elastin), Alkanolamine, Siliciumdioxid, Aluminiumoxid, weitere Metalloxide, Phosphate, wie Natriumhexametaphosphat oder Glycerin.

Bevorzugt eingesetzte partikuläre UV-Filter sind dabei:
- unbehandelte Titandioxide wie z.B. die Produkte Microtitanium Dioxide MT 500 B der Fa. Tayca; Titandioxd P25 der Fa. Degussa,
- Nachbehandelte mikronisierte Titandioxide mit Aluminiumoxid und Siliciumdioxid Nachbahandlung wie z.B. das Produkt "Microtitanium Dioxide MT 100 SA der Tayca; oder das Produkt "Tioveil Fin" der Fa. Uniqema,
- Nachbehandelte mikronisierte Titandioxide mit Aluminiumoxid und/oder Aluminiumstearate/laurate Nachbehandlung wie z.B. Microtitanium Dioxide MT 100 T der Fa. Tayca, Eusolex T-2000 der Firma Merck,
- Nachbehandelte mikronisierte Titandioxide mit Eisenoxid und/oder Eisenstearate Nachbehandlung wie z.B. das Produkt "Microtitanium Dioxide MT 100 F" der Fa. Tayca,
- Nachbehandelte mikronisierte Titandioxide mit Siliciumdioxide, Aluminiumoxid und Silicon Nachbehandlung wie z.B. das Produkt "Microtitanium Dioxide MT 100 SAS",der Fa. Tayca,
- Nachbehandelte mikronisierte Titandioxide mit Natrumhexameta¬phosphate, wie z.B. das Produkt "Microtitanium Dioxide MT 150 W" der Fa. Tayca.

Die zur Kombination eingesetzten behandelten mikronisierten Titandioxide können auch nachbehandelt sein mit:
- Octyltrimethoxysilane; wie z.B. das Produkt Tego Sun T 805 der Fa. Degussa,
- Siliciumdioxid; wie z.B. das Produkt Parsol T-X der Fa. DSM,
- Aluminiumoxid und Stearinsäure; wie z.B. das Produkt UV-Titan M160 der Fa. Sachtleben,
- Aluminium und Glycerin; wie z.B. das Produkt UV-Titan der Fa. Sachtleben
- Aluminium und Silikonölen, wie z.B. das Produkt UV-Titan M262 der Fa. Sachtleben,
- Natriumhexamethaphosphat und Polyvinylpyrrolidon,
- Polydimethylsiloxane, wie z.B. das Produkt 70250 Cardre UF TiO2SI3" der Fa. Cardre,
- Polydimethylhydrogensiloxane, wie z.B. das Produkt Microtitanium Dioxide USP Grade Hydrophobic" der Fa. Color Techniques.

Ferner kann auch die Kombination mit folgenden Produkten vorteilhaft sein:
- Unbehandelte Zinkoxide wie z. B. das Produkt Z-Cote der Fa. BASF (Sunsmart), Nanox der Fa. Elementis
- Nachbehandelte Zinkoxide wie z.B die folgenden Produkte:
   - "Zinc Oxide CS-5" der Fa. Toshibi (ZnO nachbehandelt mit polymethylhydrogenosiloxane)
   - Nanogard Zinc Oxide FN der Fa. Nanophase Technologies
   - "SPD-Z1" der Fa Shin-Etsu (ZnO nachbehandelt mit einem Silikongepfropften Acrylpolymer, dispergiert in Cyclodimethylsiloxane
   - "Escalol Z100" der Fa ISP (Aluminiumoxid nachbehandeltes ZnO dispergiert in einer ethylhexyl methoxycinnamate/PVP-hexadecene/ methicone copolymer Mischung)
   - "Fuji ZNO-SMS-10" der Fa. Fuji Pigment (ZnO nachbehandelt mit Siliciumdioxid und Polymethylsilesquioxan);
   - Unbehandeltes Ceroxide Mikropigment z.B. mit der Bezeichnung "Colloidal Cerium Oxide" der Fa Rhone Poulenc
   - Unbehandelte und/oder nachbehandelte Eisenoxide mit der Bezeichnung Nanogar der Fa. Arnaud.

Beispielhaft können auch Mischungen verschiedener Metalloxide , wie z.B. Titandioxid und Ceroxid mit und ohne Nachbenhandlung eingesetzt werden, wie z.B. das Produkt Sunveil A der Fa. Ikeda. Außerdem können auch Mischungen von Aluminiumoxid, Siliciumdioxid und Silikonnachbehandeltem Titandioxid, Zinkoxid-Mischungen wie z.B. das Produkt UV-Titan M261 der Fa. Sachtleben verwendet werden.

Diese anorganischen UV-Filter werden in der Regel in einer Menge von 0,1 Gewichtsprozent bis 25 Gewichtsprozent, vorzugsweise 2 Gew.-% bis 10 Gew.-%, in die Zubereitungen eingearbeitet.

Durch Kombination von einer oder mehrerer der genannten Verbindungen mit UV-Filterwirkung kann die Schutzwirkung gegen schädliche Einwirkungen der UV-Strahlung optimiert werden.

Alle genannten UV-Filter können auch in verkapselter Form eingesetzt werden. Insbesondere ist es von Vorteil organische UV-Filter in verkapselter Form einzusetzen.

Dabei sind die Kapseln in erfindungsgemäß einzusetzenden Zubereitungen vorzugsweise in solchen Mengen enthalten, die gewährleisten, dass die verkapselten UV-Filter in den oben angegebenen Gewichtsprozentverhältnissen in der Zubereitung vorliegen.

In den beschriebenen Zubereitungen, die den erfindungsgemäßen Extrakt enthalten, können weiterhin auch Farbpigmente enthalten sein, wobei der Schichtaufbau der Pigmente nicht limitiert ist.

Vorzugsweise sollte das Farbpigment bei Einsatz von 0,5 bis 5 Gew.-% hautfarben oder bräunlich sein. Die Auswahl eines entsprechenden Pigments ist für den Fachmann geläufig.

Bevorzugte Zubereitungen können ebenfalls mindestens einen weiteren kosmetischen Wirkstoff enthalten, beispielsweise ausgewählt aus Antioxidantien, Anti-Ageing-, Anti-Falten-, Anti-Schuppen-, Anti-Akne-, Anti-Cellulite-Wirkstoffen, Deodorants oder Vitaminen.

Die schützende Wirkung von Zubereitungen gegen oxidativen Stress bzw. gegen die Einwirkung von Radikalen kann verbessert werden, wenn die Zubereitungen ein oder mehrere Antioxidantien enthalten, wobei es dem Fachmann keinerlei Schwierigkeiten bereitet geeignet schnell oder zeitverzögert wirkende Antioxidantien auszuwählen.

Es gibt viele aus der Fachliteratur bekannte und bewährte Substanzen, die als Antioxidantien verwendet werden können, z.B. Aminosäuren (z.B. Glycin, Histidin, Tyrosin, Tryptophan) und deren Derivate, Imidazole, (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall-) Chelatoren, (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA, Pentasodium ethylenediamin tetramethylen phosphonat und deren Derivate, ungesättigte Fettsäuren und deren Derivate, Vitamin C und Derivate (z.B. Ascorbylpalmitat, Magnesium-Ascorbylphosphat, Ascorbylacetat), Tocopherole und Derivate (z.B. Vitamin-E-acetat), Vitamin A und Derivate (z.B. Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordohydroguajaretsäure, Trihydroxybutyrophenon, Quercitin, Harnsäure und deren Derivate, Mannose und deren Derivate, Zink und dessen Derivate (z.B. ZnO, ZnSO₄), Selen und dessen Derivate (z.B. Selen-methionin), Stilbene und deren Derivate (z.B. Stilbenoxid, trans-Stilbenoxid).

Geeignete Antioxidantien sind auch Verbindungen der Formeln A oder B 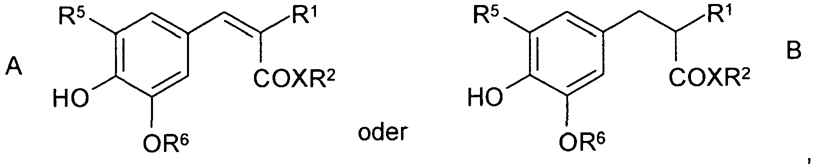 worin
R¹ aus der Gruppe -C(O)CH₃, -CO₂R³, -C(O)NH₂ und -C(O)N(R⁴)₂ ausgewählt werden kann,
X O oder NH,
R² lineares oder verzweigtes Alkyl mit 1 bis 30 C-Atomen,
R³ lineares oder verzweigtes Alkyl mit 1 bis 20 C-Atomen,
R⁴ jeweils unabhängig voneinander H oder lineares oder verzweigtes Alkyl mit 1 bis 8 C-Atomen,
R⁵ H, lineares oder verzweigtes Alkyl mit 1 bis 8 C-Atomen oder lineares oder verzweigtes Alkoxy mit 1 bis 8 C-Atomen und
R⁶ lineares oder verzweigtes Alkyl mit 1 bis 8 C-Atomen bedeutet, vorzugsweise Derivate der 2-(4-Hydroxy-3,5-dimethoxybenzyliden)-malonsäure und/oder 2-(4-Hydroxy-3,5-dimethoxybenzyl)-malonsäure, besonders bevorzugt 2-(4-Hydroxy-3,5-dimethoxybenzyliden)-malonsäure-bis-(2-ethylhexyl)ester (z.B. Oxynex® ST Liquid) und/oder 2-(4-Hydroxy-3,5-dimethoxybenzyl)-malonsäure-bis-(2-ethylhexyl)ester (z.B. RonaCare® AP).

Mischungen von Antioxidantien sind ebenfalls zur Verwendung in den erfindungsgemäßen Zubereitungen geeignet. Bekannte und käufliche Mischungen sind beispielsweise Mischungen enthaltend als aktive Inhaltsstoffe Lecithin, L-(+)-Ascorbylpalmitat und Zitronensäure, natürtiche Tocopherole, L-(+)-Ascorbylpalmitat, L-(+)-Ascorbinsäure und Zitronensäure (z.B. Oxynex® K LIQUID), Tocopherolextrakte aus natürlichen Quellen, L-(+)-Ascorbylpalmitat, L-(+)-Ascorbinsäure und Zitronensäure (z.B. Oxynex® L LIQUID), DL-α-Tocopherol, L (+)-Ascorbylpalmitat, Zitronensäure und Lecithin (z.B. Oxynex® LM) oder Butylhydroxytoluol (BHT), L-(+)-Ascorbylpalmitat und Zitronensäure (z.B. Oxynex® 2004). Derartige Antioxidantien werden in den erfindungsgemäßen Zusammensetzungen üblicherweise in Mengen von 0,1 bis 20 Gew.-%, bevorzugt in Mengen von 0,1 bis 10 Gew.-% eingesetzt.

Unter den Phenolen, die erfindungsgemäß verwendet werden können, sind die teilweise als Naturstoffe vorkommenden Polyphenole für Anwendungen im pharmazeutischen, kosmetischen oder Ernährungsbereich besonders interessant. Beispielsweise weisen die hauptsächlich als Pflanzenfarbstoffe bekannten Flavonoide oder Bioflavonoide häufig ein antioxidantes Potential auf. Mit Effekten des Substitutionsmusters von Mono- und Dihydroxyflavonen beschäftigen sich K. Lemanska, H. Szymusiak, B. Tyrakowska, R. Zielinski, I.M.C.M. Rietjens; Current Topics in Biophysics 2000, 24(2), 101-108. Es wird dort beobachtet, dass Dihydroxyflavone mit einer OH-Gruppe benachbart zur Ketofunktion oder OH Gruppen in 3'4'-oder 6,7- oder 7,8-Position antioxidative Eigenschaften aufweisen, während andere Mono- und Dihydroxyflavone teilweise keine antioxidativen Eigenschaften aufweisen.

Häufig wird Quercetin (Cyanidanol, Cyanidenolon 1522, Meletin, Sophoretin, Ericin, 3,3',4',5,7-Pentahydroxyflavon) als besonders wirksames Antioxidans genannt (z.B. C.A. Rice-Evans, N.J. Miller, G. Paganga, Trends in Plant Science 1997, 2(4), 152-159). K. Lemanska, H. Szymusiak, B. Tyrakowska, R. Zielinski, A.E.M.F. Soffers und I.M.C.M. Rietjens (Free Radical Biology&Medicine 2001, 31(7), 869-881 untersuchen die pH-Abhängigkeit der antioxidanten Wirkung von Hydoxyflavonen. Über den gesamten pH-Bereich zeigt Quercetin die höchste Aktivität der untersuchten Strukturen.

Geeignete Anti-Ageing Wirkstoffe, insbesondere für hautpflegende Zubereitungen, sind vorzugsweise sogenannte kompatible Solute. Es handelt sich dabei um Substanzen, die an der Osmoregulation von Pflanzen oder Mikroorganismen beteiligt sind und aus diesen Organismen isoliert werden können. Unter den Oberbegriff kompatible Solute werden dabei auch die in der Deutschen Patentanmeldung DE-A-10133202 beschriebenen Osmolyte gefasst. Geeignete Osmolyte sind beispielsweise die Polyole, Methylamin- Verbindungen und Aminosäuren sowie jeweils deren Vorstufen. Als Osmolyte werden im Sinne der Deutschen Patentanmeldung DE-A-10133202 insbesondere Substanzen aus der Gruppe der Polyole, wie beispielsweise myo-Inositol, Mannitol oder Sorbitol und/oder einer oder mehrere der nachfolgend genannten osmolytisch wirksamen Stoffe verstanden: Taurin, Cholin, Betain, Phosphorylcholin, Glycerophosphorylcholine, Glutamin, Glycin, α-Alanin, Glutamat, Aspartat, Prolin, und Taurin. Vorstufen dieser Stoffe sind beispielsweise Glucose, Glucose-Polymere, Phosphatidylcholin, Phosphatidylinositol, anorganische Phosphate, Proteine, Peptide und Polyaminsäuren. Vorstufen sind z. B. Verbindungen, die durch metabolische Schritte in Osmolyte umgewandelt werden.

Vorzugsweise werden erfindungsgemäß als kompatible Solute Substanzen gewählt aus der Gruppe bestehend aus Pyrimidincarbonsäuren (wie Ectoin und Hydroxyectoin), Prolin, Betain, Glutamin, cyclisches Diphosphoglycerat, N.-Acetylornithin, Trimethylamine-N-oxid Di-myo-inositol-phosphat (DIP), cyclisches 2,3-diphosphoglycerat (cDPG), 1,1-Diglycerin-Phosphat (DGP), ß-Mannosylglycerat (Firoin), ß-Mannosylglyceramid (Firoin-A) oder/und Di-mannosyl-di-inositolphosphat (DMIP) oder ein optisches Isomer, Derivat, z.B. eine Säure, ein Salz oder Ester dieser Verbindungen oder Kombinationen davon eingesetzt.

Dabei sind unter den Pyrimidincarbonsäuren insbesondere Ectoin ((S)-1,4,5,6-Tetrahydro-2-methyl-4-pyrimidincarbonsäure) und Hydroxyectoin ((S,S)-1,4,5,6-Tetrahydro-5-hydroxy-2-methyl-4-pyrimidincarbonsäure) und deren Derivate zu nennen.

Additional können als Anti-Ageing Wirkstoffe Produkte der Firma Merck wie z.B. 5,7-Dihydroxy-2-methyl-chromon, vermarktet unter dem Handelsnamen RonaCare®Luremine, Ronacare®Isoquercetin, Ronacare®Tilirosid oderRonacare®Cyclopeptide 5 verwendet werden.

Die einzusetzenden Zubereitungen können als weitere Inhaltsstoffe Vitamine enthalten. Bevorzugt sind Vitamine und Vitamin-Derivate ausgewählt aus Vitamin A, Vitamin-A-Propionat, Vitamin-A-Palmitat, Vitamin-A-Acetat, Retinol, Vitamin B, Thiaminchloridhydrochlorid (Vitamin B₁), Riboflavin (Vitamin B₂), Nicotinsäureamid, Vitamin C (Ascorbinsäure), Vitamin D, Ergocalciferol (Vitamin D₂), Vitamin E, DL-α-Tocopherol, Tocopherol-E-Acetat, Tocopherolhydrogensuccinat, Vitamin K₁, Esculin (Vitamin P-Wirkstoff), Thiamin (Vitamin B₁), Nicotinsäure (Niacin), Pyridoxin, Pyridoxal, Pyridoxamin, (Vitamin B₆), Panthothensäure, Biotin, Folsäure und Cobalamin (Vitamin B₁₂), insbesondere bevorzugt Vitamin-A-Palmitat, Vitamin C und dessen Derivate, DL-α-Tocopherol, Tocopherol-E-Acetat, Nicotinsäure, Pantothensäure und Biotin. Vitamine werden mit den flavonoidhaltigen Vormischungen oder Zubereitungen üblicherweise bei kosmetischer Anwendung in Bereichen von 0,01 bis 5,0 Gew.-%, bezogen auf das Gesamtgewicht, zugesetzt. Ernährungsphysiologische Anwendungen orientieren sich am jeweiligen empfohlenen Vitaminbedarf.

Die beschriebenen Retinoide sind gleichzeitig auch wirksame Anti-Cellulite-Wirkstoffe. Ein ebenfalls bekannter Anti-Cellulite-Wirkstoff ist Koffein.

Gegenstand der vorliegenden Erfindung ist auch ein Verfahren zur Herstellung einer Zubereitung, wie zuvor beschrieben, dadurch gekennzeichnet, dass der Extrakt aus Pflanzenteilen von *Darlingtonia californica* mit einem für topische Anwendungen geeigneten Träger vermischt wird. Optional kann die Vermischung auch mit weiteren Hilfs- und/ oder Füllstoffen erfolgen. Geeignete Trägerstoffe sowie Hilfs- oder Füllstoffe sind im nachfolgenden Teil ausführlich beschrieben.

Die genannten Bestandteile der Zubereitung können in der üblichen Weise eingearbeitet werden, mit Hilfe von Techniken, die dem Fachmann wohl bekannt sind.

Die kosmetischen und dermatologischen Zubereitungen können in verschiedenen Formen vorliegen. So können sie z. B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsion, beispielsweise vom Typ Waser-in-Öl-in-Wasser (W/O/W) oder O/W/O, ein Gel, einen festen Stift, eine Salbe oder auch ein Aerosol darstellen. Bevorzugt werden Emulsionen. O/W-Emulsinen werden besonders bevorzugt. Emulsionen, W/O-Emulsionen und O/W-Emulsionen sind in üblicher Weise erhältlich.

Als Anwendungsform der einzusetzenden Zubereitungen seien z.B. genannt: Lösungen, Suspensionen, Emulsionen, PIT-Emulsionen, Pasten, Salben, Gele, Cremes, Lotionen, Puder, Seifen, tensidhaltige Reinigungspräparate, Öle, Aerosole, Pflaster, Umschläge, Verbände und Sprays.

Bevorzugte Hilfsstoffe stammen aus der Gruppe der Konservierungsstoffe, Stabilisatoren, Lösungsvermittler, Färbemittel, Geruchsverbesserer.

Salben, Pasten, Cremes und Gele können die üblichen Trägerstoffe enthalten, die für die topische Verabreichung geeignet sind, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Puder und Sprays können die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen leichtflüchtigen, verflüssigten Treibmittel, z.B. Chlorfluorkohlenwasserstoffe, Propan/Butan oder Dimethylether, enthalten. Auch Druckluft ist vorteilhaft zu verwenden.

Lösungen und Emulsionen können die üblichen Trägerstoffe wie Lösungsmittel, Lösungsvermittler und Emulgatoren, z.B. Wasser, Ethanol, Isopropanol, Ethylcarbonat, Ethylacetat, Benzylalkohol, Benzylbenzoat, Propylenglykol, 1,3 Butylglykol, Öle, insbesondere Baumwollsaatöl, Erdnussöl, Maiskeimöl, Olivenöl, Rizinusöl und Sesamöl, XTend 226 (L'Oréal), Glycerinfettsäureester, Polyethylenglykole und Fettsäureester des Sorbitans oder Gemische dieser Stoffe enthalten.

Ein bevorzugter Lösungsvermittler generell ist 2-Isopropyl-5-methyl-cyclohexancarbonyl-D-Alaninmethylester.

Suspensionen können die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Seifen können die üblichen Trägerstoffe wie Alkalisalze von Fettsäuren, Salze von Fettsäurehalbestern, Fettsäureeiweißhydrolysaten, Isothionate, Lanolin, Fettalkohol, Pflanzenöle, Pflanzenextrakte, Glycerin, Zucker oder Gemische dieser Stoffe enthalten.

Tensidhaltige Reinigungsprodukte können die üblichen Trägerstoffe wie Salze von Fettalkoholsulfaten, Fettalkoholethersulfaten, Sulfobernsteinsäurehalbestern, Fettsäureeiweißhydrolysaten, Isothionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Gesichts- und Körperöle können die üblichen Trägerstoffe wie synthetische Öle wie Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Weitere typische kosmetische Anwendungsformen sind auch Lippenstifte, Lippenpflegestifte, Puder-, Emulsions- und Wachs-Make up sowie Sonnenschutz-, Prä-Sun- und After-Sun-Präparate.

Zu den bevorzugten Zubereitungsformen gehören insbesondere auch Emulsionen.

Emulsionen sind vorteilhaft und enthalten z. B. die genannten Fette, Öle, Wachse und anderen Fettkörper, sowie Wasser und einen Emulgator, wie er üblicherweise für einen solchen Typ der Zubereitung verwendet wird.

Die Lipidphase kann vorteilhaft gewählt werden aus folgender Substanzgruppe:
- Mineralöle, Mineralwachse
- Öle, wie Triglyceride der Caprin oder der Caprylsäure, ferner natürtiche Öle wie z. B. Rizinusöl;
- Fette, Wachse und andere natürtiche und synthetische Fettkörper, vorzugsweise Ester von Fettsäuren mit Alkoholen niedriger C Zahl, z.B. mit Isopropanol, Propylenglykol oder Glycerin, oder Ester von Fettalkoholen mit Alkansäuren niedriger C Zahl oder mit Fettsäuren;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigtem und/oder ungesättigten, verzweigten und/oder unverzweigten Alkancarbonsäuren einer Kettenlänge von 3 bis 30 C Atomen und gesättigten und/oder ungesättigten, verzweigten und/oder unverzweigten Alkoholen einer Kettenlänge von 3 bis 30 C Atomen, aus der Gruppe der Ester aus aromatischen Carbonsäure und gesättigten und/oder ungesättigten, verzweigten und/oder unverzweigten Alkoholen einer Kettenlänge von 3 bis 30 C Atomen. Solche Esteröle können dann vorteilhaft gewählt werden aus der Gruppe Isopropylmyristat, Isopropylpalmitat, Isopropylstearat, Isopropyloleat, n Butylstearat, n-Hexyllaurat, n Decyloleat, Isooctylstearat, Isononylstearat, Isononylisononanoat, 2 Ethylhexylpalmitat, 2 Ethylhexyllaurat, 2 Hexaldecylstearat, 2 Octyldodecylpalmitat, Oleyloleat, Oleylerucat, Erucyloleat, Erucylerucat sowie synthetische, halbsynthetische und natürliche Gemische solcher Ester, z. B. Jojobaöl.

Ferner kann die Ölphase vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12-18 C Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Die wässrige Phase der einzusetzenden Zubereitungen enthält gegebenenfalls vorteilhaft Alkohole, Diole oder Polyole niedriger C Zahl, sowie deren Ether, vorzugsweise Ethanol, Isopropanol, Propylenglykol, Glycerin, Ethylenglykol, Ethylenglykolmonoethyl oder -monobutylether, Propylenglykolmonomethyl, -monoethyl oder -monobutylether, Diethylenglykolmonomethyl oder-monoethylether und analoge Produkte, ferner Alkohole niedriger C Zahl, z. B. Ethanol, Isopropanol, 1,2 Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Insbesondere werden Gemische der vorstehend genannten Lösemittel verwendet. Bei alkoholischen Lösemitteln kann Wasser ein weiterer Bestandteil sein.

In einer bevorzugten Ausführungsform enthalten die einzusetzenden Zubereitungen hydrophile Tenside. Die hydrophilen Tenside werden bevorzugt gewählt aus der Gruppe der Alkylglucoside, der Acyllactylate, der Betaine sowie der Cocoamphoacetate.

Als Emulgatoren können beispielsweise die bekannten W/O- und O/W-Emulgatoren verwendet werden. Es ist vorteilhaft, weitere übliche Co-Emulgatoren in den bevorzugten O/W-Emulsionen zu verwenden.

Vorteilhaft werden als Co-Emulgatoren beispielsweise O/W-Emulgatoren gewählt, vornehmlich aus der Gruppe der Substanzen mit HLB-Werten von 11-16, ganz besonders vorteilhaft mit HLB-Werten von 14,5-15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkhole, Cetylalkohole, Cetylstearylalkohole (Cetearylalkohole) zu wählen.

Es ist ferner von Vorteil, die Fettsäureethoxylate aus folgender Gruppe zu wählen:
Polyethylenglycol(20)stearat, Polyethylenglycol(21)stearat,
Polyethylenglycol(22)stearat, Polyethylenglycol(23)stearat,
Polyethylenglycol(24)stearat, Polyethylenglycol(25)stearat,
Polyethylenglycol(12)isostearat, Polyethylenglycol(13)isostearat,
Polyethylenglycol(14)isostearat, Polyethylenglycol(15)isostearat,
Polyethylenglycol(16)isostearat, Polyethylenglycol(17)isostearat,
Polyethylenglycol(18)isostearat, Polyethylenglycol(19)isostearat,
Polyethylenglycol(20)isostearat, Polyethylenglycol(21)isostearat,
Polyethylenglycol(22)isostearat, Polyethylenglycol(23)isostearat,
Polyethylenglycol(24)isostearat, Polyethylenglycol(25)isostearat,
Polyethylenglycol(12)oleat, Polyethylenglycol(13)oleat,
Polyethylenglycol(14)oleat, Polyethylenglycol(15)oleat,
Polyethylenglycol(16)oleat, Polyethylenglycol(17)oleat,
Polyethylenglycol(18)oleat, Polyethylenglycol(19)oleat,
Polyethylenglycol(20)oleat.

Als ethoxylierte Alkylethercarbonsäure bzw. deren Salz kann vorteilhaft das Natriumlaureth-11-carboxylat verwendet werden. Als Alkylethersulfat kann Natrium Laureth1-4sulfat vorteilhaft verwendet werden. Als ethoxyliertes Cholesterinderivat kann vorteilhaft Polyethylenglycol(30)Cholesterylether verwendet werden. Auch Polyethylenglycol(25)Sojasterol hat sich bewährt. Als ethoxylierte Triglyceride können vorteilhaft die Polyethylenglycol(60) Evening Primrose Glycerides verwendet werden (Evening Primrose = Nachtkerze).

Weiterhin ist von Vorteil, die Polyethylenglycolglycerinfettsäureester aus der Gruppe Polyethylenglycol(20)glyceryllaurat,
Polyethylenglycol(21)glyceryllaurat, Polyethylenglycol(22)glyceryllaurat,
Polyethylenglycol(23)glyceryllaurat,
Polyethylenglycol(6)glycerylcaprat/cprinat,
Polyethylenglycol(20)glyceryloleat, Polyethylenglycol(20)glycerylisostearat,
Polyethylenglycol(18)glyceryloleat(cocoat) zu wählen.

Es ist ebenfalls günstig, die Sorbitanester aus der Gruppe
Polyethylenglycol(20)sorbitanmonolaurat,
Polyethylenglycol(20)sorbitanmonostearat,
Polyethylenglycol(20)sorbitanmonoisostearat,
Polyethylenglycol(20)sorbitanmonopalmitat,
Polyethylenglycol(20)sorbitanmonooleat zu wählen.

Als fakultative, dennoch erfindungsgemäß gegebenenfalls vorteilhafte W/O-Emulgatoren können eingesetzt werden:
Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12-18 C Atome, Diglycerinester gesättigter und/oder ungesättigter, verzweigter und/oder unverzweigter Alkancarbonsäuren einer Kettenlänge von 8 bis 24, insbesondere 12-18 C¬ Atomen, Monoglycerinether gesättigter und/oder ungesättigter, verzweigter und/oder unverzweigter Alkohole einer Kettenlänge von 8 bis 24, insbesondere 12-18 C-Atomen, Diglycerinether gesättigter und/oder ungesättigter, verzweigter und/oder unverzweigter Alkohole einer Kettenlänge von 8 bis 24, insbesondere 12-18 C Atomen, Propylenglycolester gesättigter und/oder ungesättigter, verzweigter und/oder unverzweigter Alkancarbonsäuren einer Kettenlänge von 8 bis 24, insbesondere 12-18 C-Atomen sowie Sorbitanester gesättigter und/oder ungesättigter, verzweigter und/oder unverzweigter Alkancarbonsäuren einer Kettenlänge von 8 bis 24, insbesondere 12-18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat oder PEG-30-dipolyhydroxystearat.

Die Zubereitung kann kosmetische Adjuvantien enthalten, welche in dieser Art von Zubereitungen üblicherweise verwendet werden, wie z.B. Verdickungsmittel, weichmachende Mittel, Befeuchtungsmittel, grenzflächenaktive Mittel, Emulgatoren, Konservierungsmittel, Mittel gegen Schaumbildung, Parfums, Wachse, Lanolin, Treibmittel, Farbstoffe und/oder Pigmente, und andere in der Kosmetik gewöhnlich verwendete Ingredienzien.

Man kann als Dispersions- bzw. Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglykol, Glycerin und Sorbit.

Eine bevorzugte Ausführungsform der Erfindung ist eine Emulsion, welche als Schutzcreme oder -milch vorliegt und beispielsweise Fettalkohole, Fettsäuren, Fettsäureester, insbesondere Triglyceride von Fettsäuren, Lanolin, natürliche und synthetische Öle oder Wachse und Emulgatoren in Anwesenheit von Wasser enthält.

Weitere bevorzugte Ausführungsformen stellen ölige Lotionen auf Basis von natürlichen oder synthetischen Ölen und Wachsen, Lanolin, Fettsäureestern, insbesondere Triglyceriden von Fettsäuren, oder öligalkoholische Lotionen auf Basis eines Niedrigalkohols, wie Ethanol, oder eines Glycerols, wie Propylenglykol, und/oder eines Polyols, wie Glycerin, und Ölen, Wachsen und Fettsäureestern, wie Triglyceriden von Fettsäuren, dar.

Die Zubereitung kann auch als alkoholisches Gel vorliegen, welches einen oder mehrere Niedrigalkohole oder -polyole, wie Ethanol, Propylenglykol oder Glycerin, und ein Verdickungsmittel, wie Kieselerde umfasst. Die öligalkoholischen Gele enthalten außerdem natürliches oder synthetisches Öl oder Wachs.
Die festen Stifte bestehen aus natürlichen oder synthetischen Wachsen und Ölen, Fettalkoholen, Fettsäuren, Fettsäureestern, Lanolin und anderen Fettkörpern.

Ist eine Zubereitung als Aerosol konfektioniert, verwendet man in der Regel die üblichen Treibmittel, wie Alkane, Fluoralkane und Chlorfluoralkane , bevorzugt Alkane.
Auch ohne weitere Ausführungen wird davon ausgegangen, dass ein Fachmann die obige Beschreibung in weitestem Umfang nutzen kann. Die bevorzugten Ausführungsformen und Beispiele sind deswegen lediglich als beschreibende, keinesfalls als in irgendeiner Weise limitierende Offenbarung aufzufassen. Die Beispiele sollen die vorliegende Erfindung näher erläutern, ohne deren Umfang zu beschränken.

### Beispiele:

### Beispiel 1: Ethanolextrakt aus Darlingtonia californica

2,8 g Schlauchblätter von *Darlingtonia californica* werden bei 40°C und 200mbar im Vakuumtrockenschrank zur Gewichtskonstanz getrocknet, grob zerkleinert und mit 250 ml Ethanol unter Rückfluss extrahiert und abfiltriert. Nach Entfernen des Lösungsmittels werden 497 mg Extrakt erhalten.

### Beispiel 2: Durchführung des B16 V Maus-Melanomzellen-Tests:

B16V Maus-Melanomzellen (Hersteller: DSMZ; Artikel-Nr,: ACC370) werden in RPMI- Medium (Invitrogen, Artikel-Nr.: 31870), dem zusätzlich noch 10% FBS (Fetal Bovine Serum; Invitrogen, Artikel-Nr: 10499044), 2mM L-Glutamin (Invitrogen, Artikel-Nr: 25030) und 1mM Natriumpyruvat (Invitrogen, Artikel-Nr:11360) hinzugefügt werden, überführt und 72h bei 37°C und 5%CO₂ inkubiert. Das Medium wird abgetrennt und die Zellen werden einmal mit 10mL DPBS (Dulbecco's Phosphate-Buffered Salines; Invitrogen, Artikel-Nr. 14190) gewaschen und das Medium anschließend abgesaugt. Es wird 1 mL HyQtase- Cell Detachment Solution (Hyclone, Artikel-Nr. SV30030.01) auf die Zellen gegeben. Die Flasche wird mehrfach geschwenkt und die HyQtase- Cell Detachment Solution anschließend durch Absaugen entfernt. Dann werden die Zellen für 5min im Inkubator bei 37°C und 5%CO₂ inkubiert. Die Zellen werden in dem modifizierten RPMI-Medium (s.o.) aufgenommen und die Zellzahl bestimmt. Dazu werden die Zellen mit Trypanblau angefärbt und in einer Neubauer-Zählkammer ausgezählt. Anschließend werden die Zellen erneut im modifizierten RPMI-Medium (s.o.) in einer definierten Zellzahl von 80000 Zellen pro Well (6 Well Clear Plate, TCT, PS (Nunc)) ausgesät.

Die Zellen werden 24h bei 37°C und 5%CO₂ inkubiert, danach das Medium entfernt. Anschließend werden 1980 µL der Substanzverdünnung zugegeben. Für diese Substanzverdünnung wird der Extrakt aus Beispiel 1 in DMSO gelöst und anschließend über einen Sterilfilter (0.2µm, Millipore, Artikel Nr. SLLG013SL) filtriert. Die Lösung wird dann mit dem modifizierten RPMI-Medium (s.o., jedoch beträgt in diesem Fall der FBS-Gehalt nur 5%) so verdünnt, dass die Endkonzentration des Extraktes 0,1 mg/ml bzw. 9,95 mg/ml beträgt.

Dann werden 20µL einer alpha-MSH Lösung (alpha-melanocyte stimulating hormone, DMSO, Sigma, Artikel-Nr.:D2650) zugegeben, so dass die alpha-MSH Konzentration im Well bei 10⁻⁸ M liegt. Anschließend wird erneut 24h bei 37°C und 5% CO₂ inkubiert. Der in diesem Abschnitt beschriebene Vorgang wird insgesamt noch zweimal wiederholt.

Nach der letzten Inkubationsperiode wird das Medium abgesaugt und die Zellen mit 1000µL DPBS (Invitrogen, Artikel-Nr. 14190) gewaschen. Das Medium wird erneut abgesaugt. Es werden 250µL HyQtase- Cell Detachment Solution (Hyclone, Artikel-Nr. SV30030.01) auf die Zellen gegeben. Die 6 Well-Platte wird mehrfach geschwenkt und die HyQtase-Cell Detachment Solution anschließend durch Absaugen entfernt. Dann werden die Zellen für 5min im Inkubator bei 37°C und 5%CO₂ inkubiert. Die Zellen werden in 1.5mL DPBS (Invitrogen, Artikel-Nr. 14190) aufgenommen und in ein Cup (SARSTEDT, Ref. 72.692.005) überführt. Anschließend wird die Zellzahl bestimmt. Dazu werden die Zellen mit Trypanblau angefärbt und in einer Neubauer-Zählkammer ausgezählt. Die Zellen bei 3500g 1 min zentrifugiert. Die erhaltenen Pellets werden photographiert und anschließend der Überstand abgesaugt. Die Pellets werden in 1mL 1N NaOH 1h bei 80°C aufgelöst und dann auf RT abgekühlt. Anschließend werden pro Cup viermal je 200µL (als Vierfachbestimmung) in eine 96 Well Platte (VWR, Artikel Nr. 4100636981) pipettiert und die Absorption bei 405nm Wellenlänge ermittelt (Safire, Tecan). Mittels einer Eichgeraden kann so der Gehalt an Melanin bestimmt werden

Als Vergleich wird parallel je eine Probe ohne Extrakt aber mit 0,1 % DMSO bzw. mit 0,1% DMSO und alpha-MSH (Konzentration im Well bei 10⁻⁸ M) verwendet.

### Ergebnisse:

Der Extrakt aus *Darlingtonia californica* aus Beispiel 1 bewirkt eine Erhöhung des Melaningehalts sowohl in den nicht stimulierten als auch in den durch α-MSH zur Bräunung stimulierten Melanocyten. Das bedeutet, dass die erfindungsgemäßen Extrakte sogar Zellen, die bereits durch α-MSH stimuliert worden sind, dazu anregen können noch zusätzlich weiter Melanin zu synthetisieren.

**Tabelle 1: Gehalt an Melanin pro Zelle:**

| | Melaningehalt [pg/Zelle] |
|---|---|
| DMSO (0,1%) | 13,4 |
| DMSO (0,1%)+α-MSH | 35,7 |
| 100 µg Extrakt aus Beispiel 1 +α-MSH | 51,8 |
| 100 µg Extrakt aus Beispiel 1 | 20,1 |

**Beispiel 3: O/W-Formulierung**

| **Bestandteile /Handelsname** | **Bezugsquelle** | **INCI** | **[Gew-%]** |
|---|---|---|---|
| **A** | | | |
| Marlipal 1618/11 | (1) | CETEARETH-11 | 3 |
| Lanette O | (2) | CETEARYLALCOHOL | 7 |
| Luvitol EHO | (3) | CETEARYLOCTANOATE | 5 |
| Tegosoft TN | (4) | C12-15 ALKYLBENZOATE | 2.5 |
| Miglyol 812 N | (1) | CAPRYLIC/CAPRIC TRIGLYCERIDE | 2.5 |
| Propyl-4-hydroxybenzoat | (5) | PROPYLPARABEN | 0.05 |
| Extrakt aus Beispiel 1 | | | 0.5 |
| | | | |
| **B** | | | |
| 1,2-Propandiol | (5) | PROPYLENE GLYCOL | 4 |
| Methyl-4-hydroxybenzoat | (5) | METHYLPARABEN | 0.15 |
| Wasser, demineralisiert | | AQUA (WATER) | ad 100 |
| | | | |
| **Gesamt** | | | **100.00** |

### Herstellungsverfahren:

Zunächst wird die Phase A auf 75°C und die Phase B auf 80 °C erwärmt. Danach wird Phase B unter Rühren langsam zu Phase A gegeben und solange gerührt, bis eine homogene Mischung entsteht.

### Bezugsquellen:

(1) Sasol Germany GmbH (2) Cognis GmbH (3) BASF AG (4) Degussa-Goldschmidt AG (5) Merck KGaA/Rona®

**Beispiel 4: O/W-Formulierung**

| **Bestandteile /Handelsname** | **Bezugsquelle** | **INCI** | **[Gew-%]** |
|---|---|---|---|
| **A** | | | |
| Marlipal 1618/11 | (1) | CETEARETH-11 | 3 |
| Lanette O | (2) | CETEARYLALCOHOL | 7 |
| Luvitol EHO | (3) | CETEARYLOCTANOATE | 5 |
| Tegosoft TN | (4) | C12-15 ALKYLBENZOATE | 2.5 |
| Miglyol 812 N | (1) | CAPRYLIC/CAPRIC TRIGLYCERIDE | 2.5 |
| Propyl-4-hydroxybenzoat | (5) | PROPYLPARABEN | 0.05 |

| **B** | | | |
|---|---|---|---|
| 1,2-Propandiol | (5) | PROPYLENE GLYCOL | 4 |
| Methyl-4-hydroxybenzoat | (5) | METHYLPARABEN | 0.15 |
| Extrakt aus Beispiel 1 | | | 1.0 |
| Wasser, demineralisiert | | AQUA (WATER) | ad 100 |
| **Gesamt** | | | **100.00** |

### Herstellungsverfahren:

Zunächst wird die Phase A auf 75°C und die Phase B auf 80 °C erwärmt. Danach wird Phase B unter Rühren langsam zu Phase A gegeben und solange gerührt, bis eine homogene Mischung entsteht.

### Bezugsquellen:

(1) Sasol Germany GmbH (2) Cognis GmbH (3) BASF AG (4) Degussa-Goldschmidt AG (5) Merck KGaA/Rona®

**Beispiel 5: O/W-Formulierung**

| **Bestandteile / Handelsname** | **Bezugsquelle** | **INCI** | **[Gew-%]** |
|---|---|---|---|
| **A** | | | |
| Tego Care 150 | (1) | GLYCERYL STEARATE, STEARETH-25, CETETH-20, STEARYL ALCOHOL | 8 |
| Lanette O | (2) | CETEARYL ALCOHOL | 1.5 |
| Luvitol EHO | (3) | CETEARYL OCTANOATE | 5 |
| Miglyol 812 N | (4) | CAPRYLIC/CAPRIC TRIGLYCERIDE | 5 |
| Paraffin liquid | (5) | PARAFFINUM LIQUIDUM (MINERAL OIL) | 3 |
| AbilWax 2434 | (1) | STEAROXY DIMETHICONE | 1.6 |
| Dow Corning 200 Fluid (350 cs) | (6) | DIMETHICONE | 0.5 |
| Propyl-4-hydroxybenzoat | (5) | PROPYLLPARABEN | 0.05 |
| | | | |

| **B** | | | |
|---|---|---|---|
| 1,2-Propandiol | (5) | PROPYLENE GLYCOL | 3 |
| Methyl-4-hydroxybenzoat | (5) | METHYLPARABEN | 0.15 |
| Wasser, entmineralisiert | | AQUA (WATER) | ad 100 |
| | | | |

| **C** | | | |
|---|---|---|---|
| Probiol L 05018 (Leere Liposomen) | (7) | AQUA, ALCOHOL DENAT, LECITHIN, GLYCERINE, DISODIUM PHOSPHATE | 5 |
| Wasser, entmineralisiert | | AQUA (WATER) | 10.00 |
| Extrakt aus Beispiel 1 | | | 1.0 |
| | | | |
| **Gesamt** | | | **100.00** |

### Herstellungsverfahren:

Zunächst werden die Phasen A und B auf 80 °C erwärmt. Danach wird Phase B unter Rühren langsam zu Phase A gegeben und homogenisiert. Dann wird abgekühlt und die Phase C bei 40°C zugegeben.

### Bezugsquellen:

(1) Degussa-Goldschmidt AG, (2) Cognis GmbH, (3) BASF AG, (4) Sasol Germany GmbH, (5) Merck KGaA/Rona®, (6) Dow Corning, (7) Kuhs GmbH & Co. KG

### Beispiel 6: W/O-Formulierung

| **Bestandteile / Handelsname** | **Bezugsquelle** | **INCI** | **[Gew-%]** |
|---|---|---|---|
| **A** | | | |
| Dow Corning 3225 C | (1) | CYCLOMETHICONE, DIMETHICONE COPOLYOL | 23.6 |
| Propyl-4-hydroxybenzoat | (2) | PROPYLPARABEN | 0.05 |
| | | | |

| **B** | | | |
|---|---|---|---|
| Extrakt aus Beispiel 1 | | | 2.0 |
| Methyl-4-hydroxybenzoat | (2) | METHYLPARABEN | 0.15 |
| 1,2-Propandiol | (2) | PROPYLENE GLYCOL | 35.9 |
| Wasser, entmineralisiert | | AQUA (WATER) | ad 100 |
| | | | |
| **Gesamt** | | | **100.00** |

### Herstellungsverfahren:

Zunächst wird die Phase B aufgelöst und dann wird sie zu Phase A gegeben. Der pH-Wert wird mit Natronlauge bzw. Citronensäure auf den Wert pH = 6.0 eingestellt.

### Bezugsquellen:

(1) Dow Corning (2) Merck KGaA/Rona®

### Beispiel 7: O/W Anti-aging Creme mit UV A/B Schutz

| **Bestandteile / Handelsname** | **Bezugsquelle** | **INCI** | **[Gew-%]** |
|---|---|---|---|
| **A** | | | |
| Eusolex® 2292 | (1) | ETHYLHEXYL METHOXYCINNAMATE, BHT | 3 |

| | | | |
|---|---|---|---|
| Eusolex® 4360 | (1) | BENZOPHENONE-3 | 0.5 |
| Tego Care 150 | (2) | GLYCERYL STEARATE, STEARETH-25, CETETH-20, STEARYL ALCOHOL | 8 |
| Lanette O | (3) | CETEARYL ALCOHOL | 1.5 |
| Luvitol EHO | (4) | CETEARYL OCTANOATE | 5 |
| Miglyol 812 N | (5) | CAPRYLIC/CAPRIC TRIGLYCERIDE | 5 |
| Paraffin liquid | (1) | PARAFFINUM LIQUIDUM (MINERAL OIL) | 3 |
| Abil-Wax 2434 | (2) | STEAROXY DIMETHICONE | 1.6 |
| Dow Corning 200 Fluid (350 cs) | (6) | DIMETHICONE | 0.5 |
| Propyl-4-hydroxybenzoat | (1) | PROPYLPARABEN | 0.05 |

| **B** | | | |
|---|---|---|---|
| 1,2-Propandiol | (1) | PROPYLENE GLYCOL | 3 |
| Methyl-4-hydroxybenzoat Natriumsalz | (1) | SODIUM METHYLPARABEN | 0.17 |
| Extrakt aus Beispiel 1 | | | 0.5 |
| Wasser, demineralisiert | | AQUA (WATER) | ad 100 |
| | | | |
| **Gesamt** | | | **100.00** |

### Herstellungsverfahren:

Zunächst werden die Phasen A und B getrennt gemischt und auf 80°C erwärmt. Danach wird Phase B langsam unter Rühren zu Phase A gegeben. Es wird homogenisiert auf Raumtemperatur abgekühlt.

Bezugsquellen: (1) Merck KGaA/Rona®, (2) Degussa-Goldschmidt AG, (3) Cognis GmbH, (4) BASF AG, (5) Sasol Germany GmbH, (6)

## Patentansprüche

1. Extrakt aus Pflanzenteilen von *Darlingtonia californica* zur Verwendung als Selbstbräunungsmittel.

2. Extrakt nach Anspruch 1, **dadurch gekennzeichnet, dass** die Pflanzenteile Blätter sind.

3. Extrakt nach Anspruch 1 oder 2 zur Steigerung der Melaninsynthese, Verbesserung des Melanintransports und/oder Verbesserung der Verteilung von Melanin in suprabasalen Schichten.

4. Zubereitung enthaltend einen Extrakt aus Pflanzenteilen von *Darlingtonia californica,* **dadurch gekennzeichnet, dass** mindestens eine weitere Selbstbräunungssubstanz enthalten ist.

5. Zubereitung nach Anspruch 4, **dadurch gekennzeichnet, dass** die Pflanzenteile Blätter sind.

6. Zubereitung nach Anspruch 4 oder 5, **dadurch gekennzeichnet, dass** die Zubereitung einen für topische Anwendungen geeigneten Träger und weitere Hilfs- oder Füllstoffe enthält.

7. Zubereitung nach einem oder mehreren der Ansprüche 4 bis 6, **dadurch gekennzeichnet, dass** die Zubereitung 0,01 bis 99 Gew.% des Extrakts, bezogen auf das Gesamtgewicht der Zubereitung, enthält.

8. Verfahren zur Herstellung einer Zubereitung nach einem oder mehreren der Ansprüche 4 bis 7, **dadurch gekennzeichnet, dass** der Extrakt aus Pflanzenteilen von *Darlingtonia californica* mit der mindestens einen weiteren Selbstbräunungssubstanz und mit dem für topische Anwendungen geeigneten Träger und den weiteren Hilfs- oder Füllstoffen vermischt wird.

## Claims

1. Extract from plant parts of *Darlingtonia californica* for use as self-tanning agent.

2. Extract according to Claim 1, **characterised in that** the plant parts are leaves.

3. Extract according to Claim 1 or 2 for increasing melanin synthesis, improving melanin transport and/or improving the distribution of melanin in suprabasal layers.

4. Preparation comprising an extract from plant parts of *Darlingtonia californica,* **characterised in that** at least one further self-tanning substance is present.

5. Preparation according to Claim 4, **characterised in that** the plant parts are leaves.

6. Preparation according to Claim 4 or 5, **characterised in that** the preparation comprises a vehicle which is suitable for topical applications and further assistants or fillers.

7. Preparation according to one or more of Claims 4 to 6, **characterised in that** the preparation comprises 0.01 to 99% by weight of the extract, based on the total weight of the preparation.

8. Process for producing a preparation according to one or more of Claims 4 to 7, **characterised in that** the extract from plant parts of *Darlingtonia californica* is mixed with the at least one further self-tanning substance and with the vehicle which is suitable for topical applications and the further assistants or fillers.

## Revendications

1. Extrait de parties de plante de *Darlingtonia californica*, pour une utilisation comme agent auto-bronzant.

2. Extrait selon la revendication 1, **caractérisé en ce que** les parties de plante sont des feuilles.

3. Extrait selon la revendication 1 ou 2, destiné à l'augmentation de la synthèse de mélanine, l'amélioration du transport de la mélanine et/ou l'amélioration de la distribution de la mélanine dans les couches supra-basales.

4. Préparation comprenant un extrait de parties de plante de *Darlingtonia californica*, **caractérisée en ce qu'**au moins une autre substance auto-bronzante est présente.

5. Préparation selon la revendication 4, **caractérisée en ce que** les parties de plante sont des feuilles.

6. Préparation selon la revendication 4 ou 5, **caractérisée en ce que** la préparation comprend un véhicule qui est convenable pour des applications topiques et d'autres auxiliaires ou charges.

7. Préparation selon l'une ou plusieurs parmi les revendications 4 à 6, **caractérisée en ce que** la préparation comprend de 0,01 à 99% en poids de l'extrait, sur la base du poids total de la préparation.

8. Procédé de production d'une préparation selon l'une ou plusieurs parmi les revendications 4 à 7, **caractérisé en ce que** l'extrait de parties de plante de *Darlingtonia californica* est mélangé avec la au moins une autre substance auto-bronzante et avec le véhicule qui est convenable pour des applications topiques et les autres auxiliaires ou charges.
